# EUROPEAN PATENT SPECIFICATION

(11) **EP 3 912 678 B1**
(45) Date of publication and mention of the grant of the patent: **15.10.2025**
(21) Application number: 21174013.9
(22) Date of filing: 17.05.2021
(51) Int. Cl.: A61K 8/35, A61K 8/42, A61K 8/64, A61K 8/27, A61K 8/49, A61K 8/9789, A61N 5/06, A61P 17/00, A61Q 7/00

(54) **KIT FOR STIMULATING HAIR GROWTH AND RESTORING SCALP BALANCE BY A COSMETIC TRICHOLOGICAL PROTOCOL**
KIT ZUR STIMULIERUNG DES HAARWACHSTUMS UND ZUR WIEDERHERSTELLUNG DES KOPFHAUTGLEICHGEWICHTS DURCH EIN KOSMETISCHES TRICHOLOGISCHES PROTOKOLL
KIT PERMETTANT DE STIMULER LA CROISSANCE DES CHEVEUX ET DE RESTAURER L'ÉQUILIBRE DU CUIR CHEVELU À L'AIDE D'UN PROTOCOLE TRICHOLOGIQUE COSMÉTIQUE

(30) Priority: 18.05.2020 IT 202000011353
(43) Date of publication of application: 24.11.2021
(73) Proprietor: Sanders S.r.l., 20124 Milano (IT)
(72) Inventor: ANGELINI, Matteo, W23DG London (GB)
(74) Representative: Rapisardi, Mariacristina

(56) References cited:
- JP-U- 3 218 397
- TW-A- 201 302 262
- ISTITUTO HELVETICO SANDERS: "Rajon Hi-Tech: una tecnologia innovativa contro la calvizie", YOUTBE, 2 September 2019 (2019-09-02), pages 5 pp., XP054981383, Retrieved from the Internet <URL:https://www.youtube.com/watch?v=8WZY3_E-huk> [retrieved on 20210208]
- DATABASE GNPD [online] MINTEL; 5 April 2019 (2019-04-05), ANONYMOUS: "Hair & Scalp Shampoo", XP055773044, retrieved from https://www.gnpd.com/sinatra/recordpage/6466791/ Database accession no. 6466791
- DATABASE GNPD [online] MINTEL; 20 January 2020 (2020-01-20), ANONYMOUS: "Burdock & Ho Shou Wu Scalp Tonic", XP055773045, retrieved from https://www.gnpd.com/sinatra/recordpage/7196049/ Database accession no. 7196049
- DATABASE GNPD [online] MINTEL; 3 September 2019 (2019-09-03), ANONYMOUS: "Leave-in Hair and Scalp Treatment", XP055773046, retrieved from https://www.gnpd.com/sinatra/recordpage/6840951/ Database accession no. 6840951
- DATABASE GNPD [online] MINTEL; 17 September 2019 (2019-09-17), ANONYMOUS: "Coffee Lotion", XP055773047, retrieved from https://www.gnpd.com/sinatra/recordpage/6868571/ Database accession no. 6868571
- DATABASE GNPD [online] MINTEL; 12 December 2016 (2016-12-12), ANONYMOUS: "Platinum Hair Growth Treatment", XP055773048, retrieved from https://www.gnpd.com/sinatra/recordpage/4479861/ Database accession no. 4479861
- DATABASE GNPD [online] MINTEL; 29 June 2015 (2015-06-29), ANONYMOUS: "Nourishing Scalp and Strand Serum", XP055773049, retrieved from https://www.gnpd.com/sinatra/recordpage/3306907/ Database accession no. 3306907
- DATABASE GNPD [online] MINTEL; 15 September 2009 (2009-09-15), ANONYMOUS: "4 Anti-Hair Loss Concentrate", XP055773050, retrieved from https://www.gnpd.com/sinatra/recordpage/1176853/ Database accession no. 1176853
- DATABASE GNPD [online] MINTEL; 28 November 2018 (2018-11-28), ANONYMOUS: "Solution", XP055773051, retrieved from https://www.gnpd.com/sinatra/recordpage/6124571/ Database accession no. 6124571
- DATABASE GNPD [online] MINTEL; 2 July 2012 (2012-07-02), ANONYMOUS: "Daily Root & Scalp Stimulator", XP055773052, retrieved from https://www.gnpd.com/sinatra/recordpage/1822321/ Database accession no. 1822321
- DATABASE GNPD [online] MINTEL; 4 June 2018 (2018-06-04), ANONYMOUS: "Concentrate Hair Lotion", XP055773053, retrieved from https://www.gnpd.com/sinatra/recordpage/5668949/ Database accession no. 5668949
- DATABASE GNPD [online] MINTEL; 8 October 2015 (2015-10-08), ANONYMOUS: "Elixir", XP055773043, retrieved from https://www.gnpd.com/sinatra/recordpage/3409617/ Database accession no. 3409617
- DATABASE GNPD [online] MINTEL; 18 April 2018 (2018-04-18), ANONYMOUS: "Hair Growth Lotion", XP055632931, retrieved from https://www.gnpd.com/sinatra/recordpage/5598249/ Database accession no. 5598249

## Description

The present invention relates to a kit for stimulating hair growth and restoring scalp balance by a cosmetic trichological protocol.

The present invention relates to the aesthetic, cosmetic and phytocosmetic sector and, more precisely, the sector of natural compositions for treating hair and the scalp, in particular in cases of abundant and premature hair loss, miniaturization and destructurization of the shafts, in the presence of seborrhoea, dandruff and redness of the scalp.

### Prior art

The scalp is the area of the skin on which the hair is located. As it is made of biological material subject to alterations, abnormalities, disorders or actual diseases, the scalp has, like all the integumentary apparatus, the task of offering protection and heat regulation. Hair also increases the protection and heat regulation actions, and has an important role in social and sexual communication.

The health of the scalp is closely related to the wellbeing and growth of hair. In fact, the hair follicles, which are the organs that produce the hair, are located therein.

The problem of hair loss is very widespread and affects various people, both men and women, also conditioning their quality of life and interpersonal relations.

When faced with a problem of baldness, or thinning, the first thing to do is observe the scalp and understand its state of health.

The most common abnormalities affecting the scalp are often associated with itchiness, flaking, dandruff and seborrhoea.

There are different cosmetic trichological treatments on the market that aim to reduce the problems listed above.

In the excerpt from the video published by Istituto Helvetico Sanders: "Rajon Hi-Tech: una tecnologia innovativa contro la calvizie". Youtube. 2 September 2019 (retrieved from the Internet: URL:https://www.youtube.com/watch?v= 8WZY3) discloses an apparatus and a method for treating the hair and scalp against hair loss. JP 3218397 U discloses a hair growth assisting device having a light irradiating portion for irradiating light onto a scalp. TW 201302232 A discloses a device for treating baldness and promoting hair growth comprising: simultaneously using two LED light sources, 660 nm red light and 940 nm near-infrared light to illuminate the head and neck.

The task of the present invention is that of providing a kit for stimulating hair growth and restoring scalp balance, which enables the drawbacks of the prior art described above to be solved.

Within the scope of this technical task, an aim of the present invention is that of providing a kit for stimulating hair growth and restoring scalp balance that can be easily used at home by the person presenting trichological problems.

Another aim of the invention is that of providing a method for the aesthetic treatment of the hair and scalp that enables the results expected by the person to be optimized as much as possible. These aims of the present invention are enabled by means of a kit for stimulating hair growth and restoring scalp balance according to Claim1.

Current invention also discloses a method for the non-therapeutic aesthetic treatment of the hair and scalp of a person according to Claim 13.

Other main aspects of the invention are reported in the following dependent claims.

Further characteristics and advantages of the invention will more fully emerge from the description of a preferred but not exclusive embodiment of the kit for stimulating hair growth and restoring scalp balance by a cosmetic trichological protocol illustrated by way of non-limiting indication in the accompanying drawings, in which:
- figure 1 shows a plan view from below of the helmet illustrating the arrangement of the first diodes which emit infra-red light and the second diodes which emit red light based on the male thinning pattern;
- figure 2 shows the plan view of the helmet of figure 1 wherein the arrows indicate the gradual activation direction of a part of the first diodes from a central area of the helmet to peripheral areas of the helmet.

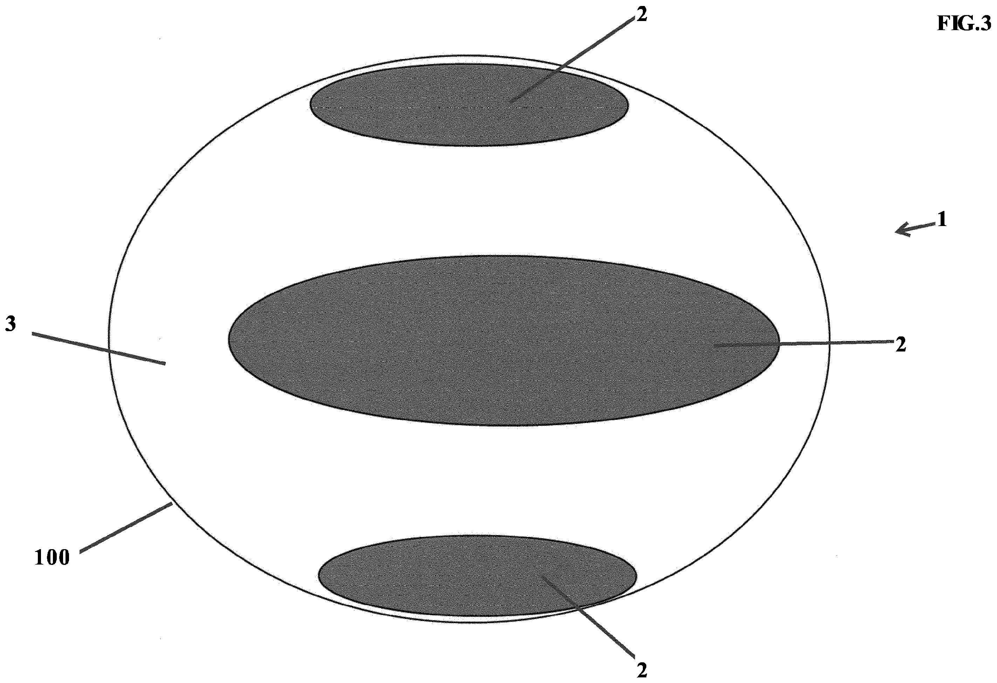

- figure 3 shows a plan view from below of the helmet illustrating the arrangement of the first diodes which emit infra-red light and the second diodes which emit red light based on the female thinning pattern;
- figure 4 shows the plan view of the helmet of figure 3 wherein the arrows indicate the gradual activation direction of a part of the first diodes from a central area of the helmet to peripheral areas of the helmet.

### Skin-correcting Treatment

The skin-correcting treatment is used to correct the alterations present both at scalp level and at hair follicle level.

It comprises substances with sebo-normalizing, keratolytic, anti-inflammatory, decongestant, anti-itching, soothing, nutritional, hydrating, detoxifying and anti-oxidizing properties.

The results that come from the use of such complexes are:
- normalization of the scalp: seborrheic states, redness, flaking and dandruff.
- elimination of the symptoms associated with unbalanced skin such as itchiness, scalp dysesthesia and burning.
- purification of the follicular environment
- Correct and regular trophism of regrowth
- Thickening of the shafts
- normalization of hair loss.

The skin-correcting treatment comprises the following products:

| Product | Constituents | Advantages |
|---|---|---|
| RO Complex | Zinc PCA: sebo-normalizing, antimicrobial, keratolytic, disinfectant and weakly anti-androgenic. | Specific for dandruff and flaking. It eliminates itchiness, redness and burning. It normalizes seborrhoea. |
| | Panthenol: soothing, nourishing, hydrating and healing. | |
| | Caffeine: stimulates the micro-circulation, stimulates cellular and follicular metabolism. | It normalizes hair loss. |
| | | It thickens hair and regrowth and purifies the follicles. |
| | Camphor: revulsive, rubefacient, refreshing, anti-itching, antimicrobial, soothing and weakly analgesic. | |
| | Rosemary: purifying, sebo-normalizing, antioxidant, anti-inflammatory, anti-dandruff. | |
| | Marigold: healing, anti-inflammatory, antibacterial, antifungal and antiviral. | |
| UD Complex | Zinc PCA: sebo-normalizing, antimicrobial, keratolytic, disinfectant and weakly anti-androgenic. | It is suitable for all scalp problems, especially associated with seborrhoea. |
| | Panthenol: soothing, nourishing, hydrating and healing. | |
| | | It normalizes seborrhoea. |
| | Caffeine: stimulates the micro-circulation, stimulates cellular and follicular metabolism. | |
| | | It eliminates dandruff, flaking, itchiness, redness and burning. |
| | Camphor: revulsive, rubefacient, refreshing, anti-itching, antimicrobial, soothing and weakly analgesic. | |
| | | It thickens hair and regrowth. |
| | Urtica dioica: strongly exfoliating, anti-androgenic, anti-dandruff, sebo-normalizing, nourishing. | It normalizes hair loss. |
| | Tussilago Farfara: anti-inflammatory, decongestant, emollient, soothing, purifying and antiseptic. | It purifies the follicles. |
| CP Complex | Zinc PCA: sebo-normalizing, antimicrobial, keratolytic, disinfectant and weakly anti-androgenic. | It is suitable for all cases of male and female pattern hair loss. |
| | Panthenol: soothing, nourishing, hydrating and healing. | It normalizes seborrhoea. |
| | Caffeine: stimulates the micro-circulation, stimulates cellular and follicular metabolism. | It normalizes hair loss. |
| | | It eliminates dandruff, itchiness, redness and burning. |
| | Camphor: revulsive, rubefacient, refreshing, anti-itching, antimicrobial, soothing and weakly analgesic. | |
| | | It thickens hair and regrowth. |
| | Curcubita pepo: powerful anti-androgen. | It purifies the follicles. |
| | Leontopodium alpinum or edelweiss: antiox, anti-inflammatory, stimulates the microcirculation, soothing, antiseptic, skin purifying | |
| RP Complex | Zinc PCA: sebo-normalizing, antimicrobial, keratolytic, disinfectant and weakly anti-androgenic. | Particularly suitable for thinning. |
| | | It hydrates, restructures and nourishes damaged, destructured and split hair. |
| | Panthenol: soothing, nourishing, hydrating and healing. | |
| | Caffeine: stimulates the micro-circulation, stimulates cellular and follicular metabolism. | |
| | | It thickens hair and regrowth. |
| | Camphor: revulsive, rubefacient, refreshing, anti-itching, antimicrobial, soothing and weakly analgesic. | It purifies the follicles. |
| | Lily: emollient, soothing, anti-inflammatory and strongly hydrating. | |
| EG Complex | Zinc PCA: sebo-normalizing, antimicrobial, keratolytic, disinfectant and weakly anti-androgenic. | Suitable as preparatory treatment for the intervention. |
| | Panthenol: soothing, nourishing, hydrating and healing. | It promotes repair of injuries and the healing process. |
| | Caffeine: stimulates the micro-circulation, stimulates cellular and follicular metabolism. | |
| | | It normalizes seborrhoea. |
| | Camphor: revulsive, rubefacient, refreshing, anti-itching, antimicrobial, soothing and weakly analgesic. | It eliminates dandruff, itchiness, redness and burning. |
| | Eucalyptus globulus: antibacterial, refreshing, antiseptic, healing, soothing. | It thickens hair and regrowth. |
| | | It normalizes hair loss. |
| | | It purifies the follicles. |
| PRE Complex | Zinc PCA: sebo-normalizing, antimicrobial, keratolytic, disinfectant and weakly anti-androgenic. | Suitable as preparatory phase for transplantation. |
| | Panthenol: soothing, nourishing, hydrating and healing. | It normalizes seborrhoea. |
| | Caffeine: stimulates the micro-circulation, stimulates cellular and follicular metabolism. | It normalizes hair loss. It eliminates dandruff, flaking, itchiness, redness and burning. |
| | Camphor: revulsive, rubefacient, refreshing, anti-itching, antimicrobial, soothing and weakly analgesic. | |
| | | It thickens hair and regrowth. |
| | Urtica dioica: strongly exfoliating, anti-androgenic, anti-dandruff, sebo-normalizing, nourishing. | It purifies the follicles. |
| | Tussilago Farfara: anti-inflammatory, decongestant, emollient, soothing, purifying and antiseptic. | |
| | Hamamelis: analgesic, antiphlogistic, emollient. | |

### Skin-stimulating treatment

Skin-stimulating treatments aim to stimulate the necessary follicular metabolism for promoting hair growth both in terms of length and thickness.

The skin-stimulating treatment comprises the following products:

| Product | Constituents | Advantages |
|---|---|---|
| SK Complex | Zinc PCA: sebo-normalizing, antimicrobial, keratolytic, disinfectant and weakly anti-androgenic. | Particularly suitable for male and female pattern hair loss. |
| | Panthenol: soothing, nourishing, hydrating and healing. | It normalizes seborrhoea. |
| | Caffeine: stimulates the micro-circulation, stimulates cellular and follicular metabolism. | It normalizes hair loss. |
| | | It eliminates dandruff, itchiness, redness and burning. |
| | Camphor: revulsive, rubefacient, refreshing, anti-itching, antimicrobial, soothing and weakly analgesic. | |
| | | It thickens hair and regrowth. |
| | Hydrolyzed wheat proteins: strongly hydrating, nourishing and softening. | It purifies the follicles. |
| | Serenoa repens fruit extracts: powerful anti-androgen. | |
| | Vitamin C: toning, stimulates the microcirculation. | |
| | Calamus aromaticus root extracts: anti-dandruff, anti-redness, anti-inflammation, anti-bacterial and anti-fungal properties. | |
| AC Complex | Zinc PCA: sebo-normalizing, antimicrobial, keratolytic, disinfectant and weakly anti-androgenic. | Particularly suitable for thinning. |
| | | It nourishes and tones hair. |
| | Panthenol: soothing, nourishing, hydrating and healing. | |
| | Caffeine: stimulates the micro-circulation, stimulates cellular and follicular metabolism. | It thickens hair and regrowth. |
| | Camphor: revulsive, rubefacient, refreshing, anti-itching, antimicrobial, soothing and weakly analgesic. | It purifies the follicles. |
| | Vitamin A: nourishing, anti-ox. | |
| | Vitamin C: toning, stimulates the microcirculation, healing. | |
| PTA Complex | Zinc PCA: sebo-normalizing, antimicrobial, keratolytic, disinfectant and weakly anti-androgenic. | Suitable as post-transplant treatment. |
| | | It maintains the balance of the scalp. |
| | Panthenol: soothing, nourishing, hydrating and healing. | |
| | | It continues to normalize loss. |
| | Caffeine: stimulates the micro-circulation, stimulates cellular and follicular metabolism. | |
| | | It promotes the maintenance of hair on the scalp. |
| | Camphor: revulsive, rubefacient, refreshing, anti-itching, antimicrobial, soothing and weakly analgesic. | |
| | Curcubita pepo seed extracts: powerful anti-androgen. | |
| | Red clover flower extracts: antibacterial, anti-inflammatory, soothing. Powerful antiox (three times greater than vitamin C). It is a powerful natural anti-androgen. | |
| | Acetyl tetrapeptide-3: in synergy with red clover it amplifies the direct anti-androgenic action on the hair follicle. | |
| PTB Complex | Zinc PCA: sebo-normalizing, antimicrobial, keratolytic, disinfectant and weakly anti-androgenic. | Suitable for post-operation. |
| | | It maintains the balance of the scalp. |
| | Panthenol: soothing, nourishing, | |
| | hydrating and healing. | It continues to normalize loss. |
| | Caffeine: stimulates the micro-circulation, stimulates cellular and follicular metabolism. | |
| | | It promotes the maintenance of hair on the scalp. |
| | Camphor: revulsive, rubefacient, refreshing, anti-itching, antimicrobial, soothing and weakly analgesic. | |
| | | It promotes the healing of injuries. |
| | Hydrolyzed wheat proteins: strongly hydrating, nourishing and softening. Vitamin C: toning, stimulates the microcirculation, healing. | It stimulates the adaptation of the follicles transplanted in the new environment. |
| | Arginine: accelerates healing processes and has a nourishing action. | It promotes quicker growth of transplanted hair. |
| | Blueweed seed oil: anti-inflammatory and strongly hydrating. | |
| | | It maintains and amplifies the results obtained on the hair by deeply nourishing it. |
| | Sunflower seed oil: nourishing, hydrating and maintains skin elasticity. | |
| | Red clover: antibacterial, anti-inflammatory, soothing. Powerful antiox (three times greater than vitamin C). It is a powerful natural anti-androgen. | |
| | Acetyl tetrapeptide-3: in synergy with red clover it amplifies the direct anti-androgenic action on the hair follicle. | |
| | Cardiospermum: | |
| | Serenoa repens: powerful anti-androgenic action. | |

### Support apparatus

In a first embodiment of the invention, in order to increase the receptive capacity of the hair follicles and increase the action of the treatment itself, according to the present invention, the treatment program provides for the concomitant use of the support apparatus, called Rajon.

The programmed use of such a sophisticated piece of apparatus at home guarantees maximum efficacy of the treatment for the patient.

The Rajon is made for associating the treatment with the liquid compositions according to a precise pattern. In fact, according to the present invention, the invisible light emitted (infra-red) by the first mouse is applied to the whole dry scalp before the application of the single treatment dose as it promotes vasodilation and improved skin permeability for the purpose of maximum absorption of the product. The red light emitted by the second mouse is instead used only following the application of the single treatment dose for exploiting the mechanical thrust action of the product in the intrafollicular environment.

The rajon acts through the skin massage of two mice that emit at a precise wavelength through numerous diodes. In particular, the first mouse to be used emits in the infra-red field (I.R. 905 nm) and the second in the visible field (Red 630 nm).

The methodical alternation of these wavelengths on the scalp, in association with the liquid formulation treatment, enables the following objectives to be reached:
- promoting blood supply by stimulating the cutaneous peripheral microcirculation.
- regularizing cellular activity.
- stimulating cellular metabolism and energy production.
- increasing cutaneous absorption.
- detoxifying and antibacterial activity.
- local analgesic action
- anti-oedema action

In a second embodiment of the invention the support apparatus comprises a helmet 100 on whose internal surface the first diodes 3 are positioned, configured for the emission of light in the infra-red field and the second diodes 2 configured for the emission of light in the visible field.

In particular the second diodes 2 configured to emit light in the visible field are laser diodes. Advantageously, the first diodes 3 configured to emit light in the infra-red field are distributed across the internal surface of the helmet 100 whereas the second diodes 2 configured to emit light in the visible field are distributed congruently with a thinning pattern.

In fact, men and women have different thinning patterns. In particular, in men thinning affects the temples, the front area and the vertex. In women, on the other hand, thinning affects the central parting, the parietal area and the vertex.

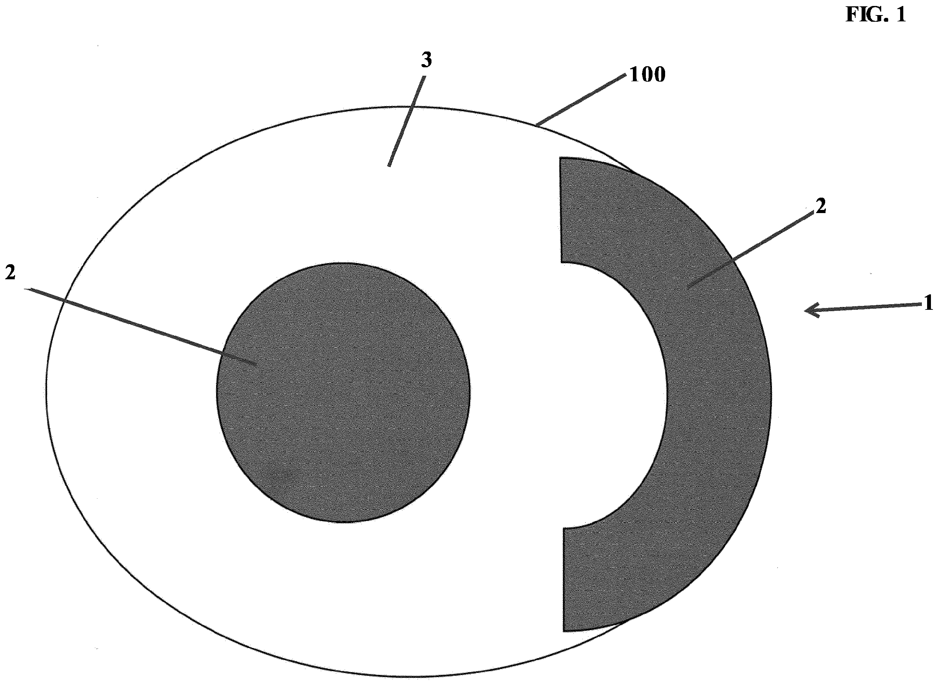

In male patients, as illustrated in figure 1, the second diodes 2 configured to emit light in the visible field will be positioned in the helmet 100 at the areas subject to thinning, i.e. the temples, the front area and the vertex.

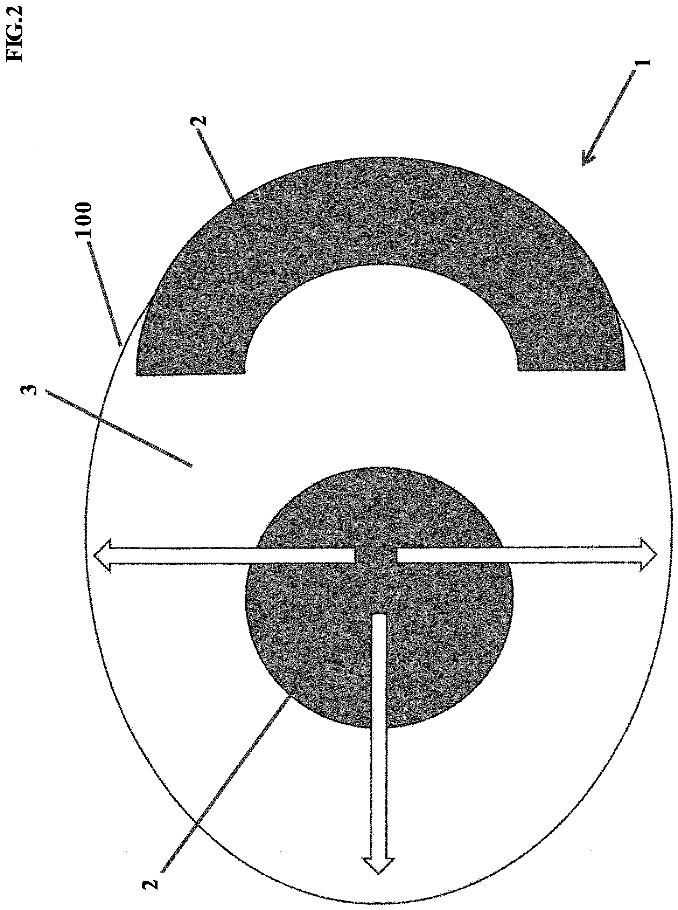

In female patients, as illustrated in figure 2, the second diodes 2 configured to emit light in the visible field will be positioned in the helmet 100 at the areas subject to thinning, i.e. the central parting, the parietal area and the vertex.

The helmet 100 is completely personalizable. The personalizations can be performed:
- based on the sex of the patient and based on the thinning pattern
- based on a predefined size (S, M, L, XL)
- based on the cranial conformation of each patient following moulding that enables the construction of a support made of transparent material with a personal 3D mould
- based on internal strings that can be regulated using a handle
- based on soft silicone tubes to be inserted along the profile of the helmet which, based on their different thickness, personalize the fit thereof.

Advantageously, the helmet 100 is of the wireless type. This enables the patient to have free hands while using it and enables him/her to move conveniently from one room to another.

The helmet 100 further comprises a rechargeable autonomous power supply source.

The helmet 100 has an on/off button through which the user can select the predefined treatment programs.

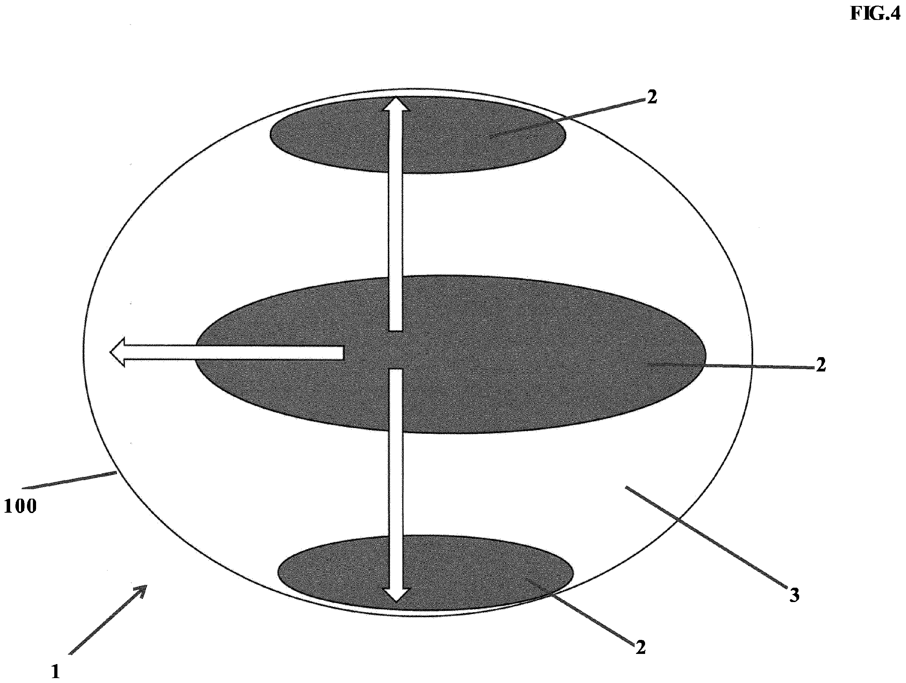

The predefined programs are as follows:
- DETOX program: duration 10 minutes, only infra-red LED activation. The LEDs are switched on consecutively starting from the centre of the helmet in 3 different directions, as illustrated by the arrows of figures 4 and 6, and finish in the three lymph drainage points, i.e. behind the ears and on the occipital part.
- CORRECTION/HAIRLOSS program: duration 20 minutes, of which 10 minutes for the activation of the infra-red LEDs and 10 minutes for the activation of the LASER LEDs.
- THICKENING/STIMULATION program: duration 20 minutes, of which 5 minutes for the activation of the infra-red LEDs and 15 minutes for the activation of the LASER LEDs.

According to the invention, there is also the possibility to load a fourth program which is also personalized for the specific user of the helmet.

Once selected, the programs will be visible through switching on a series of light indicators positioned close to the on/off button. In particular, there are 1 to 4 luminous indicators.

Finally, the helmet 100 has an interface configured to receive and transmit data and to receive energy.

In particular, an electronic device can be provided (e.g. a smartphone or PC) having an application associated with the helmet that can be consulted both by the patient and by the doctors so as to monitor the correct use of the helmet. In fact, through a relevant program, the application can record the use times, the days and also the programs used.

Through the application it will also be possible to upload software updates, including new specific/personalized use programs, which come from research and development studies.

The helmet 100 is provided with a sound emission device which is able to emit a signal at the start and the end of the treatment.

The helmet 100 has a controller (not shown in the figure) configured to selectively activate said first diodes 3 and said second diodes 2.

By way of non-limiting example, in a first actuation mode of the invention, the controller selectively activates said first diodes 3 before the application of the first or second composition and said second diodes 2 after the application of said first or second single dose composition.

In a second embodiment of the invention the controller selectively activates said first diodes 3 and said second diodes 2 before the application of said first or second single dose composition.

Advantageously, the controller is programmed with a first simultaneous activation program of said first diodes 3 and with a second sequential activation program of a part of said first diodes 3 from a central area of the helmet to peripheral areas of the helmet corresponding to lymph drainage areas of the head, in particular the ears and nape of the neck as shown in fig. 2 and 4. According to a preferred embodiment of the invention, the kit for the home treatment of the wellbeing of the skin and hair within a cosmetic trichological protocol is represented in particular by the association of support apparatus (Rajon/helmet) with the alternation of the following treatments: Skin-correcting UD complex and skin-stimulating SK Complex.

Such treatments all have a single common basis comprising the complex: zinc PCA, caffeine, panthenol and camphor.

The common components to the two formulations form this tetra complex and operate in synergy conferring:
- on the scalp an antimicrobial and sebo-regulating action, activating the microcirculation, the hydration and reepithelialization function.
- on the hair shaft a hair loss prevention and thickening action.

In the skin-correcting UD complex treatment, the compounds of the common base are also associated with extracts of urtica dioica leaves and Tussilago Farfara leaves.

Such leaf extracts give the complex stronger corrective and antimicrobial action and are very effective in people affected by seborrhoea, dandruff and severe hair loss.

In particular:
- Urtica Dioica is a grassy plant typical of temperate areas. The capacity of nettles to act as a valid remedy against hair loss comes from the fact that the roots contain beta-sitosterol, a plant sterol able to contrast the activity of dihydrotestosterone, a biologically active metabolite of the hormone testosterone, and one of the primary factors of baldness. In fact, dihydrotestosterone binds to the piliferous bulb, atrophying it. Nettles are also effective in the elimination of dandruff.
- Tussilago farfara: a perennial herbaceous plant belonging to the daisy family with anti-inflammatory, decongestant and emollient properties. The action of farfara extract reduces inflammation and oxidative stress.

In the skin-stimulating SK complex treatment, the compounds of the common base are also associated with serenoa repens fruit extracts, calamus aromaticus root extracts and hydrolized wheat proteins. These extracts make the skin-stimulating SK complex specific for cases of alopecia, also androgenetic and seborrheic, and promote the trophism and beauty of the shaft and the skin balance.

In particular:
- Serenoa repens: it is a dwarf fan palm belonging to the Arecaceae family. It has an antagonist effect on sexual hormones. Serenoa fruits contain beta-sitosterol, which inhibits the enzyme 5-alpha-reductase (5-AR) involved in the transformation of testosterone into dihydrotestosterone (DHT). In particular, beta-sitosterol blocks the isoform of 5-AR both of type I and type II. Blocking the type I isoform which is located particularly at the sebocytes, serenoa acts by reducing sebaceous secretion and therefore seborrhoea. It has also been demonstrated that the phytocomplex also acts in the direct cytoplasmic and nuclear pathway, binding the DHT receptors and preventing the hormone from performing its normal functions.
- Calamus aromaticus: it is a plant in the Araceae family. A good source of bioactive compounds, it acts as an antioxidant. It has anti-dandruff, anti-redness, anti-inflammation, anti-bacterial and anti-fungal properties.
- Hydrolyzed wheat proteins: they are plant peptides obtained from the enzymatic hydrolysis of wheat flour and constitute a hydrating complex that can penetrate into the skin structures. The proteins are comprised of amino acids such as glutamic acid, leucine, proline, arginine and glycine, which are analogous to those that normally constitute the structure of the hair. Such proteins have a revitalizing, repairing and restructuring action on hair.

The non-therapeutic aesthetic treatment of the hair and scalp according to the present invention comprises the following steps:
- a first step of radiating the scalp by means of the support apparatus which emits infra-red light for increasing the permeability of the skin, regularizing cellular metabolism and activating the local blood circulation,
- a second step of applying a first or a second single treatment dose that alternates a skin-correcting with a skin-stimulating action;
- a third step of radiating the scalp by means of the support apparatus which emits infra-red light for increasing the penetration and distribution of said first or second single dose applied.

The non-therapeutic aesthetic treatment method according to an embodiment of the invention is described below.

Dry scalp massage with the rajon which emits infra-red rays.

The infra-red massage is used to increase the permeability of the skin, regularize cellular metabolism and activate local blood microcirculation. Perform a 10-minute massage: Massage the whole head with very slow and continuous movements. The massage is to be performed from the forehead to the nape of the neck, including the sides, attempting to trace parallel lines on the scalp.

Once the scalp has been activated by the massage, apply the liquid treatment in single doses, e.g. 10 ml. The single skin-correcting and skin-stimulating doses are to be alternated 3 times a week. The sealed single dose will be opened and distributed on the scalp from the front to the vertex performing a circulatory massage. The massage must not rub the scalp but press on the skin, kneading it on the skull by making rotary movements. The first dose is the skin-correcting single dose, then in the subsequent applications it is alternated with the skin-stimulating single dose.

Once the liquid treatment has been distributed, perform a 10-minute massage with the rajon which emits red rays. This time the action is more mechanical to facilitate the penetration and distribution of the product just applied. Massage the whole head with very slow and continuous movements. The massage is to be performed from the forehead to the nape of the neck, including the sides, attempting to trace parallel lines on the scalp.

Subsequently, for at least 8 hours the head must not be washed, the treatment remains acting on the scalp which must then be washed within 24 hours of application through a complete hygiene step.

According to the present invention, the complete hygiene step envisages washing through pre-shampoo, shampoo and after-shampoo.

The hygiene step is characterized by the use of a trio of basic products which have a detergent and corrective action with which a series of products with a more aesthetic function are associated.

The hygiene step is a deep cleansing and is aimed at both the scalp and the hair. It is fundamental in order to obtain maximum efficacy by the skin-correcting treatment and the skin-stimulating treatment and therefore to maximize the results in aesthetic terms.

The pre-shampoo is the specific detergent for the scalp and preliminarily drains all the scalp impurities of both an exogenous and endogenous nature.

It exerts 4 important actions: sebo-normalizing, keratolytic, anti-inflammatory and hydrating.

The hygiene step comprises the following pre-shampoo products:

| Product | Constituents | Advantages |
|---|---|---|
| Alodermin | Zinc PCA: sebo-normalizing, antimicrobial, keratolytic, disinfectant and weakly anti-androgenic. | It is indicated for all scalp problems, even those in the acute phase. |
| | Panthenol: soothing, nourishing, hydrating and healing. | It normalizes scalp alterations such as dandruff, flaking and seborrhoea. |
| | Aloe: hydrating, calming, anti-inflammatory, disinfectant and broad-spectrum antimicrobial. | |
| | | It eliminates itchiness, redness and burning. |
| | Horse chestnut: anti-inflammatory | |
| | Horsetail: reinforcing and keratolytic due to the presence of silica. | It nourishes and reinforces hair. |
| | Chamomile: soothing, calming, anti-itching, anti-inflammatory. | |
| Pentadermin | Zinc PCA: sebo-normalizing, antimicrobial, keratolytic, disinfectant and weakly anti-androgenic. | Particularly indicated for dry and sensitive skin and destructured and damaged hair. |
| | Panthenol: soothing, nourishing, hydrating and healing. | |
| | | It normalizes scalp alterations such as dandruff, flaking and seborrhoea. |
| | Aloe: hydrating, calming, anti-inflammatory, disinfectant and broad-spectrum antimicrobial. | |
| | Pentavitin: hydrating, protective and repairing. | It eliminates itchiness, redness and burning. |
| | Echinacea: elasticizing, re-epithelizing, healing, anti-inflammatory and anti-redness. | It nourishes and reinforces hair. |
| | | It elasticizes and hydrates a dry scalp. |
| | | It hydrates and nourishes dry and destructured hair. |

The shampoo is instead the cleanser of the capillary mass.

Its function is that of completing the cleansing of the scalp and cleaning the shafts.

The table below shows the 4 types of shampoo with highly specific actions:

| Product | Constituents | Advantages |
|---|---|---|
| Allantosil | Zinc PCA: sebo-normalizing, antimicrobial, keratolytic, disinfectant and weakly anti-androgenic. | Particularly indicated for treating dandruff and flaking problems. |
| | Panthenol: soothing, nourishing, hydrating and healing. | It eliminates itchiness, redness and burning. |
| | Allantoin: skin-repairing, re-epithelizing, strongly hydrating. | It normalizes seborrhoea. |
| | Bamboo: (contains silicon) exfoliating, emollient, refreshing, toning and hydrating. | It hydrates the hair and scalp. |
| | Burdock: antiseptic, anti-inflammatory and antiox | It gives hair strength and body. |
| Allantosil volume | Zinc PCA: sebo-normalizing, antimicrobial, keratolytic, disinfectant and weakly anti-androgenic. | Particularly indicated for situations in which it is important to provide volume and hydration. |
| | Panthenol: soothing, nourishing, | |
| | hydrating and healing. | It hydrates the scalp and hair. |
| | Allantoin: skin-repairing, re-epithelizing, strongly hydrating. Bamboo: (contains silicon) exfoliating, | |
| | | It gives hair shine and body. |
| | emollient, refreshing, toning and hydrating. | It gives all the head of hair volume and softness. |
| | Burdock: antiseptic, anti-inflammatory and antioxidant Hydrolyzed wheat proteins: strongly hydrating, nourishing and softening. | |
| | | It normalizes seborrhoea. |
| | | It eliminates itchiness, redness and burning. |
| | | It eliminates dandruff and flaking. |
| Zincdermil | Zinc PCA: sebo-normalizing, antimicrobial, keratolytic, disinfectant and weakly anti-androgenic. | Particularly indicated for normalizing seborrhoea. |
| | Panthenol: soothing, nourishing, hydrating and healing. | It eliminates dandruff and flaking. |
| | Horsetail: reinforcing and keratolytic due to the presence of silica. | It eliminates itchiness, redness and burning. |
| | Lily: emollient, soothing, anti-inflammatory and strongly hydrating. | It strongly hydrates hair. |
| Amidermil | Zinc PCA: sebo-normalizing, antimicrobial, keratolytic, disinfectant and weakly anti-androgenic. | It is the shampoo indicated after the transplant operation. |
| | Panthenol: soothing, nourishing, hydrating and healing. | It specifically eliminates dandruff and flaking. |
| | Allantoin: skin-repairing, re-epithelizing, strongly hydrating. | It eliminates itchiness, redness and burning. |
| | Echinacea: elasticizing, re-epithelizing, healing, anti-inflammatory and anti-redness. | It normalizes seborrhoea. |
| | | It promotes the epithelium healing and repair processes. |
| | Amisoft: gentle surfactant. Junglans regia: keratinizing, anti-inflammatory, antiseptic, re-epithelizing, regenerating, elasticizing, healing and anti-reddening. | |
| | | It gives the head of hair softness and volume. |
| | | It is also suitable for frequent washes. |

The tonic is instead an after-shampoo which is applied to the scalp or hair according to whether the tonic is disinfectant or restructuring.

To perform its efficacy as best as possible it should not be rinsed after its application.

The following table shows the types of tonics with the related functions.

| Product | Constituents | Advantages |
|---|---|---|
| Enoxyl tonic | Zinc PCA: sebo-normalizing, antimicrobial, keratolytic, disinfectant and weakly anti-androgenic. | It is a powerful decongestant and disinfectant. |
| | Panthenol: soothing, nourishing, hydrating and healing. Caffeine: stimulates the micro-circulation, stimulates cellular and in particular follicular metabolism. | It immediately eliminates dandruff, itchiness, redness and burning. |
| | | It normalizes seborrhoea. |
| | Horsetail: reinforcing and keratolytic due to the presence of silica. | |
| | | It eliminates dandruff and flaking. |
| | Evening primrose: emollient, hydrating, decongestant, regenerating and restructuring, weak anti-androgenic. Peppermint: refreshing, toning, purifying, antiseptic and anti-itching. | |
| | | It provides an immediate sensation of skin relief. |
| | | By stimulating the micro-circulation it promotes the penetration of the active ingredients. |
| Provital Tonic | Zinc PCA: sebo-normalizing, antimicrobial, keratolytic, disinfectant and weakly anti-androgenic. | Particularly indicated for destructured and damaged hair. |
| | Panthenol: soothing, nourishing, hydrating and healing. | It gives hair shine and body. |
| | Caffeine: stimulates the micro-circulation, stimulates cellular and in particular follicular metabolism. | It improves the structure of the hair. |
| | Lily: emollient, soothing, anti-inflammatory and strongly hydrating. | |
| | Goji berries: contain vitamins, minerals and antiox substances. | |

Tonics are applied after removing excess water directly to the scalp (as in the case of Enoxyl Tonic) or especially on the end part of the capillary lengths (as in the case of Provital Tonic).

### HYGIENE STEP APPLICATION PROTOCOL

a. PRE-SHAMPOO: With the help of a micropipette, or a syringe without a needle, deposit the contents of 2 caps full of pre-shampoo on the dry scalp. The first cap must be massaged onto all of the nape of the neck and the sides, the second cap onto the whole of the upper area. The use of the micropipette, or the syringe, is so that the product is not dispersed onto the hair and for only washing the scalp when it is dry. The pre-shampoo is a skin detergent, which drains all the impurities. It is massaged for 2 minutes and then rinsed with plenty of water.
b. SHAMPOO: straight after the pre-shampoo 1 cap of shampoo is emulsified on the whole head of hair and then massaged and rinsed.
   Dab the hair with a towel.
c. AFTER SHAMPOO: distribute the product on the scalp and then massage the residue onto the shafts. There is no need to rinse.

The particular combination of skin-correcting treatments and skin-stimulating treatments with the aid of the Rajon or the helmet, and the subsequent application of the hygiene step, ensure that the person has deep cleansing of the scalp as well as a normalization thereof, which helps to prevent scalp imperfections (such as dandruff, seborrhoea, redness, etc.) often a concomitant cause of hair loss.

## Claims

1. A kit for stimulating hair growth and restoring scalp balance by a cosmetic trichological protocol, **characterized in that** it comprises at least a first and a second single dose composition comprising zinc PCA, caffeine, panthenol and camphor and support apparatus (1) having first diodes (3) configured to emit infra-red light and second diodes (2) configured to emit light in the visible field, wherein said apparatus (1) has a controller configured to actively select said first diodes (3) and said second diodes (2) and wherein said first single dose composition further comprises at least urtica dioica and Tussilago Farfara leaves, and said second single dose composition further comprises at least hydrolized wheat proteins, serenoa repens fruit extracts and calamus aromaticus root extracts.

2. The kit for stimulating hair growth and restoring scalp balance by a cosmetic trichological protocol according to the preceding claim, characterized that said first composition is of skin-correcting type and further comprises rosemary leaf extracts, marigold flower extracts, curcubita pepo seed extracts, Leontopodium alpinum flower extracts, lily bulb extracts, eucalyptus globulus leaf extracts and hamamelis leaf extracts as skin-correcting agents.

3. The kit for stimulating hair growth and restoring scalp balance by a cosmetic trichological protocol according to the preceding claim is **characterized in that** said second composition is of the skin-stimulating type and further comprises vitamin C, vitamin A, red clover flower extracts, curcubita pepo seed extracts, acetyl tetrapeptide 3, arginine, blueweed seed oil and sunflower seed oil as skin-stimulating agents.

4. The kit for stimulating hair growth and restoring scalp balance by a cosmetic trichological protocol according to claim 1 **characterized in that** said first diodes (3) emit light on a 905 nm wavelength.

5. The kit for stimulating hair growth and restoring scalp balance by a cosmetic trichological protocol according to claim 1, **characterized in that** said second diodes (2) emit light on a 630 nm wavelength.

6. The kit for stimulating the growth of the hair and restoring scalp balance by a cosmetic trichological protocol according to claim 1 **characterized in that** said support apparatus comprises a helmet (100) on the inside surface of which said first (3) and second (2) diodes are positioned.

7. The kit for stimulating hair growth and restoring scalp balance by a cosmetic trichological protocol according to the preceding claim **characterized in that** said second diodes (2) configured to emit light in the visible field are laser diodes.

8. The kit for stimulating hair growth and restoring scalp balance by a cosmetic trichological protocol according to claims 6 and 7 **characterized in that** said first diodes (3) configured to emit light in the infra-red field are distributed across the entire inside surface of the helmet (100) and **in that** said second diodes (2) configured to emit light in the visible field are distributed congruently with a thinning pattern.

9. The kit for stimulating hair growth and restoring scalp balance by a cosmetic trichological protocol according to any one of the preceding claims **characterized in that** said controller is programmed with a first program for the simultaneous activation of said first diodes (3) and with a second program for the sequential activation of a part of said first diodes (3) from a central area of the helmet to peripheral zones of the helmet corresponding to lymph drainage areas of the head.

10. The kit for stimulating hair growth and restoring scalp balance by a cosmetic trichological protocol according to claims 6 to 8 **characterized in that** said first helmet (100) is wireless.

11. The kit for stimulating hair growth and restoring scalp balance by a cosmetic trichological protocol according to claims 6 to 9 **characterized in that** said helmet (100) comprises a rechargeable autonomous power supply source.

12. The kit for stimulating hair growth and restoring scalp balance by a cosmetic trichological protocol according to claims 6 to 10 **characterized in that** said helmet (100) has an interface configured to receive and transmit data and to receive energy.

13. A method for the non-therapeutic aesthetic treatment of the hair and scalp of a person comprising a first step of radiating the scalp by means of a support apparatus (1) that emits infra-red light to increase cutaneous permeability, regulate cell metabolism and activate local blood microcirculation, a second step of applying a first or second single dose composition that alternates skin-correcting action with a skin-stimulating action, and a third step of radiating the scalp by means of a support apparatus that emits light in the red area to promote the penetration and distribution of said applied first or second single dose composition, **characterized in that** at the end of the third step the application of said first or second single dose composition has to be left to set for at least 8 hours, **in that** said first and a second single dose composition comprising zinc PCA, caffeine, panthenol and camphor and **in that** said first single dose composition further comprises at least urtica dioica and Tussilago Farfara leaves, and said second single dose composition further comprises at least hydrolized wheat proteins, serenoa repens fruit extracts and calamus aromaticus root extracts.

14. The method for the non-therapeutic aesthetic treatment of the hair and scalp according to the preceding claim, **characterized in that** said first and third radiation step are performed for 10 minutes.

## Patentansprüche

1. Kit zur Anregung des Haarwachstums und zur Wiederherstellung des Gleichgewichts der Kopfhaut durch ein kosmetisches trichologisches Protokoll, **dadurch gekennzeichnet, dass** es mindestens eine erste und eine zweite Einzeldosis-Zusammensetzung umfasst, die Zink-PCA, Koffein, Panthenol und Kampfer enthalten, sowie ein Unterstützungsgerät (1), das erste Dioden (3) zum Aussenden von Infrarotlicht und zweite Dioden (2) zum Aussenden von Licht im sichtbaren Bereich aufweist, wobei das Gerät (1) über einen Regler verfügt, der konfiguriert ist, um die ersten Dioden (3) und die zweiten Dioden (2) gezielt zu aktivieren, und wobei die erste Einzeldosis-Zusammensetzung enthält darüber hinaus mindestens Blätter von Urtica dioica (Brennnessel) und Tussilago farfara (Huflattich), und die zweite Einzeldosis-Zusammensetzung enthält darüber hinaus mindestens hydrolysierte Weizenproteine, Extrakte aus den Früchten von Serenoa repens (Sägepalme) und Extrakte aus der Wurzel von Acorus calamus (Kalmus).

2. Das Kit zur Anregung des Haarwachstums und zur Wiederherstellung des Gleichgewichts der Kopfhaut gemäß dem vorhergehenden Anspruch, **dadurch gekennzeichnet, dass** die erste Zusammensetzung ist vom hautkorrigierenden Typ und umfasst außerdem Rosmarinblattextrakte, Ringelblumenblütenextrakte, Kürbiskernextrakte (Cucurbita pepo), Edelweißblütenextrakte (Leontopodium alpinum), Lilienzwiebelextrakte, Eukalyptusblattextrakte (Eucalyptus globulus) und Hamamelisblattextrakte als hautkorrigierende Wirkstoffe

3. Das Kit zur Anregung des Haarwachstums und zur Wiederherstellung des Gleichgewichts der Kopfhaut gemäß dem vorhergehenden Anspruch, **dadurch gekennzeichnet, dass** Die zweite Zusammensetzung ist vom hautstimulierenden Typ und umfasst außerdem Vitamin C, Vitamin A, Extrakte aus Rotklee-Blüten (Trifolium pratense), Kürbiskernextrakte (Cucurbita pepo), Acetyl-Tetrapeptid-3, Arginin, Öl aus Natternkopfsamen (Echium plantagineum) und Sonnenblumenöl als hautstimulierende Wirkstoffe.

4. Das Kit zur Anregung des Haarwachstums und zur Wiederherstellung des Gleichgewichts der Kopfhaut durch ein kosmetisches trichologisches Protokoll gemäß Anspruch 1, **dadurch gekennzeichnet, dass** die ersten Dioden (3) Licht mit einer Wellenlänge von 905 nm aussenden.

5. Das Kit zur Anregung des Haarwachstums und zur Wiederherstellung des Gleichgewichts der Kopfhaut durch ein kosmetisches trichologisches Protokoll gemäß Anspruch 1, **dadurch gekennzeichnet, dass** die zweiten Dioden (2) Licht mit einer Wellenlänge von 630 nm aussenden.

6. Das Kit zur Anregung des Haarwachstums und zur Wiederherstellung des Gleichgewichts der Kopfhaut durch ein kosmetisches trichologisches Protokoll gemäß Anspruch 1, **dadurch gekennzeichnet, dass** das Unterstützungsvorrichtung ein Helmelement (100) umfasst, an dessen Innenfläche die ersten (3) und zweiten (2) Dioden angeordnet sind.

7. Das Kit zur Anregung des Haarwachstums und zur Wiederherstellung des Gleichgewichts der Kopfhaut gemäß dem vorhergehenden Anspruch, **dadurch gekennzeichnet, dass** die zweiten Dioden (2), die Licht im sichtbaren Bereich aussenden, Laserdioden sind.

8. Das Kit zur Anregung des Haarwachstums und zur Wiederherstellung des Gleichgewichts der Kopfhaut gemäß den Ansprüchen 6 und 7, **dadurch gekennzeichnet, dass** die ersten Dioden (3), die Licht im Infrarotbereich aussenden, über die gesamte Innenfläche des Helms (100) verteilt sind und dass die zweiten Dioden (2), die Licht im sichtbaren Bereich aussenden, entsprechend einem Ausdünnungsmuster verteilt sind.

9. Das Kit zur Anregung des Haarwachstums und zur Wiederherstellung des Gleichgewichts der Kopfhaut gemäß einem der vorhergehenden Ansprüche, **dadurch gekennzeichnet, dass** der Controller mit einem ersten Programm zur gleichzeitigen Aktivierung der ersten Dioden (3) und einem zweiten Programm zur sequentiellen Aktivierung eines Teils der ersten Dioden (3) von einem zentralen Bereich des Helms zu peripheren Zonen des Helms programmiert ist, die den Lymphabflusszonen des Kopfes entsprechen.

10. Das Kit zur Anregung des Haarwachstums und zur Wiederherstellung des Gleichgewichts der Kopfhaut gemäß den Ansprüchen 6 bis 8, **dadurch gekennzeichnet, dass** der Helm (100) kabellos ist.

11. Das Kit zur Anregung des Haarwachstums und zur Wiederherstellung des Gleichgewichts der Kopfhaut gemäß den Ansprüchen 6 bis 9, **dadurch gekennzeichnet, dass** der Helm (100) über eine wiederaufladbare autonome Energieversorgung verfügt.

12. Das Kit zur Anregung des Haarwachstums und zur Wiederherstellung des Gleichgewichts der Kopfhaut gemäß den Ansprüchen 6 bis 10, **dadurch gekennzeichnet, dass** der Helm (100) über eine Schnittstelle verfügt, die zum Empfangen und Übertragen von Daten sowie zum Energieempfang konfiguriert ist.

13. Ein Verfahren zur nichttherapeutischen ästhetischen Behandlung von Haar und Kopfhaut einer Person, umfassend einen ersten Schritt der Bestrahlung der Kopfhaut mittels eines Unterstützungsvorrichtung (1), die Infrarotlicht aussendet, um die kutane Permeabilität zu erhöhen, den Zellstoffwechsel zu regulieren und die lokale Mikrozirkulation des Blutes zu aktivieren, einen zweiten Schritt des Auftragens einer ersten oder zweiten Einzeldosis-Zusammensetzung, die abwechselnd eine hautkorrigierende und hautstimulierende Wirkung entfaltet, sowie einen dritten Schritt der Bestrahlung der Kopfhaut mit rotem Licht, um das Eindringen und die Verteilung der aufgetragenen ersten oder zweiten Einzeldosis-Zusammensetzung zu fördern, **dadurch gekennzeichnet, dass** am Ende des dritten Schritts die aufgetragene Zusammensetzung mindestens 8 Stunden einwirken muss, dass die erste und die zweite Einzeldosis-Zusammensetzung Zink-PCA, Koffein, Panthenol und Kampfer enthalten, wobei die erste Einzeldosis-Zusammensetzung ferner mindestens Blätter von Urtica dioica (Brennnessel) und Tussilago farfara (Huflattich) umfasst, und die zweite Einzeldosis-Zusammensetzung ferner mindestens hydrolysierte Weizenproteine, Extrakte aus den Früchten von Serenoa repens (Sägepalme) und Extrakte aus der Wurzel von Acorus calamus (Kalmus) enthält.

14. Das Verfahren zur nichttherapeutischen ästhetischen Behandlung von Haar und Kopfhaut gemäß dem vorhergehenden Anspruch, **dadurch gekennzeichnet, dass** der erste und dritte Bestrahlungsschritt jeweils 10 Minuten dauert.

## Revendications

1. Kit destiné à stimuler la pousse des cheveux et à rétablir l'équilibre du cuir chevelu selon un protocole trichologique cosmétique, **caractérisé en ce qu'**il comprend au moins une première et une deuxième composition unidose contenant du zinc PCA, de la caféine, du panthénol et du camphre, ainsi qu'un appareil de support (1) comprenant des premières diodes (3) configurées pour émettre une lumière infrarouge et des deuxièmes diodes (2) configurées pour émettre une lumière dans le domaine visible, ledit appareil (1) comprenant un contrôleur configuré pour activer de manière sélective lesdites premières diodes (3) et deuxièmes diodes (2), et la première composition unidose comprenant en outre au moins des feuilles d'ortie dioïque (*Urtica dioica*) et de Tussilage (*Tussilago farfara*), et la deuxième composition unidose comprenant en outre au moins des protéines de blé hydrolysées, des extraits de fruit de serenoa repens et des extraits de racine de calamus aromaticus.

2. Kit destiné à stimuler la pousse des cheveux et à rétablir l'équilibre du cuir chevelu selon la revendication précédente, **caractérisé en ce que** ladite première composition est de type correcteur cutané et comprend en outre des extraits de feuilles de romarin, des extraits de fleurs de calendula, des extraits de graines de Curcubita pepo, des extraits de fleurs de Leontopodium alpinum, des extraits de bulbe de lys, des extraits de feuilles d'eucalyptus globulus et des extraits de feuilles d'hamamélis, en tant qu'agents correcteurs cutanés.

3. Kit destiné à stimuler la pousse des cheveux et à rétablir l'équilibre du cuir chevelu selon la revendication précédente, **caractérisé en ce que** ladite deuxième composition est de type stimulant cutané et comprend en outre de la vitamine C, de la vitamine A, des extraits de fleurs de trèfle rouge, des extraits de graines de Curcubita pepo, de l'acétyl tétrapeptide-3, de l'arginine, de l'huile de graines de Echium et de l'huile de graines de tournesol, en tant qu'agents stimulants cutanés.

4. Le kit destiné à stimuler la pousse des cheveux et à rétablir l'équilibre du cuir chevelu selon un protocole trichologique cosmétique selon la revendication 1, **caractérisé en ce que** lesdites premières diodes (3) émettent une lumière d'une longueur d'onde de 905 nm.

5. Le kit destiné à stimuler la pousse des cheveux et à rétablir l'équilibre du cuir chevelu selon un protocole trichologique cosmétique selon la revendication 1, **caractérisé en ce que** lesdites deuxièmes diodes (2) émettent une lumière d'une longueur d'onde de 630 nm.

6. Le kit destiné à stimuler la pousse des cheveux et à rétablir l'équilibre du cuir chevelu selon un protocole trichologique cosmétique selon la revendication 1, **caractérisé en ce que** ledit appareil de support comprend un casque (100) dont la surface intérieure comporte lesdites premières (3) et deuxièmes (2) diodes.

7. Le kit destiné à stimuler la pousse des cheveux et à rétablir l'équilibre du cuir chevelu selon la revendication précédente, **caractérisé en ce que** lesdites deuxièmes diodes (2), configurées pour émettre de la lumière dans le spectre visible, sont des diodes laser.

8. Le kit destiné à stimuler la pousse des cheveux et à rétablir l'équilibre du cuir chevelu selon les revendications 6 et 7, **caractérisé en ce que** lesdites premières diodes (3), configurées pour émettre une lumière dans l'infrarouge, sont réparties sur l'ensemble de la surface intérieure du casque (100), et **en ce que** lesdites deuxièmes diodes (2), configurées pour émettre une lumière dans le visible, sont réparties de manière conforme à un schéma d'amincissement.

9. Le kit destiné à stimuler la pousse des cheveux et à rétablir l'équilibre du cuir chevelu selon l'une quelconque des revendications précédentes, **caractérisé en ce que** ledit contrôleur est programmé avec un premier programme pour l'activation simultanée desdites premières diodes (3), et un deuxième programme pour l'activation séquentielle d'une partie desdites premières diodes (3), depuis une zone centrale du casque vers les zones périphériques correspondant aux zones de drainage lymphatique de la tête.

10. Le kit destiné à stimuler la pousse des cheveux et à rétablir l'équilibre du cuir chevelu selon les revendications 6 à 8, **caractérisé en ce que** ledit casque (100) est sans fil.

11. Le kit destiné à stimuler la pousse des cheveux et à rétablir l'équilibre du cuir chevelu selon les revendications 6 à 9, **caractérisé en ce que** ledit casque (100) comprend une source d'alimentation autonome et rechargeable.

12. Le kit destiné à stimuler la pousse des cheveux et à rétablir l'équilibre du cuir chevelu selon les revendications 6 à 10, **caractérisé en ce que** ledit casque (100) comprend une interface configurée pour recevoir et transmettre des données et recevoir de l'énergie.

13. Procédé de traitement esthétique non thérapeutique des cheveux et du cuir chevelu d'une personne, comprenant une première étape consistant à irradier le cuir chevelu à l'aide d'un appareil de support (1) émettant une lumière infrarouge pour augmenter la perméabilité cutanée, réguler le métabolisme cellulaire et activer la microcirculation sanguine locale, une deuxième étape consistant à appliquer une première ou une deuxième composition unidose, alternant action correctrice cutanée et action stimulante cutanée, une troisième étape consistant à irradier le cuir chevelu au moyen d'un appareil de support émettant une lumière rouge afin de favoriser la pénétration et la diffusion de ladite composition appliquée, **caractérisé en ce qu'**à la fin de la troisième étape, l'application de ladite composition unidose doit rester en place pendant au moins 8 heures, **en ce que** les première et deuxième compositions unidose comprennent du zinc PCA, de la caféine, du panthénol et du camphre, et **en ce que** la première composition comprend en outre au moins des feuilles d'ortie dioïque (Urtica dioica) et de Tussilage (Tussilago farfara), et la deuxième composition comprend au moins des protéines de blé hydrolysées, des extraits de fruit de Serenoa repens et des extraits de racine de Calamus aromaticus.

14. Le procédé de traitement esthétique non thérapeutique des cheveux et du cuir chevelu selon la revendication précédente, **caractérisé en ce que** les première et troisième étapes d'irradiation sont effectuées pendant 10 minutes.
